(19) 

**Europäisches Patentamt**
**European Patent Office**
**Office européen des brevets**

(11) **EP 4 553 846 A1**

(12) **EUROPEAN PATENT APPLICATION**

(43) Date of publication:
**14.05.2025 Bulletin 2025/20**

(21) Application number: **24208579.3**

(22) Date of filing: **24.10.2024**

(51) International Patent Classification (IPC):
**G16H 40/63** (2018.01)    **G16H 40/67** (2018.01)

(52) Cooperative Patent Classification (CPC):
**G16H 40/63; G16H 40/67**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.11.2023 KR 20230154385**

(71) Applicant: **I-SENS, INC.**
**Seoul 06646 (KR)**

(72) Inventor: **KIM, Hae Na**
**06646 Seoul (KR)**

(74) Representative: **Westphal, Mussgnug & Partner Patentanwälte mbB**
**Am Riettor 5**
**78048 Villingen-Schwenningen (DE)**

(54) **ELECTRONIC APPARATUS AND METHOD OF CONTROLLING THE SAME**

(57)    Provided are an electronic apparatus and a method of controlling the same. The method includes acquiring sensor data indicating an concentration of an analyte in a host, displaying a user interface (UI) element indicating a value of the sensor data, determining whether to perform post-processing on the sensor data on the basis of the sensor data, performing post-processing on the sensor data on the basis of a result of the determination, and updating a display of the UI element on the basis of the postprocessed sensor data.

FIG.2

EP 4 553 846 A1

**Description**

CROSS-REFERENCE TO RELATED APPLICATION

This application claims priority to and the benefit of Korean Patent Application No. 10-2023-0154385, filed on November 9, 2023, the disclosure of which is incorporated herein by reference in its entirety.

BACKGROUND

**1. Field of the Invention**

The present disclosure relates to an electronic apparatus and a method of controlling the same, and more particularly, to an electronic apparatus for processing sensor data that indicates an concentration of an analyte in a host and a method of controlling the same.

**2. Discussion of Related Art**

A continuous glucose monitoring system (CGMS) is a system that uses a sensor in contact with a user's body fluid (e.g., interstitial fluid) to acquire and provide the user's blood glucose concentration to the user. The CGMS includes a continuous glucose monitor (CGM) that is attached to a user's body to detect a signal from the user's body fluid, and a user terminal device that provides a blood glucose concentration to the user.

The user terminal device provides the user's blood glucose concentration on the basis of a signal detected by the CGM, but the signal detected by the CGM may have noise. For this reason, the blood glucose concentration provided to the user may be distorted, and the user may be confused.

Therefore, a technology for processing noise in a signal measured by a CGM is necessary to avoid confusing a user.

SUMMARY OF THE INVENTION

The present disclosure is directed to providing an electronic apparatus for minimizing distortion of a user's blood glucose concentration value.

The present disclosure is also directed to providing an electronic apparatus for displaying a blood glucose concentration in a way that is convenient for a user to view.

Technical objects of the present disclosure are not limited to those described above, and other technical objects that have not been described above will be clearly understood by those of ordinary skill in the art from the following description.

According to an aspect of the present disclosure, there is provided a method of controlling an electronic apparatus, which includes acquiring sensor data indicating an concentration of an analyte in a host, displaying a user interface (UI) element indicating a value of the sensor data, determining whether to perform post-processing on the sensor data on the basis of the sensor data, performing post-processing on the sensor data on the basis of a result of the determination, and updating a display of the UI element on the basis of the postprocessed sensor data.

The determining of whether to perform the post-processing may include, when a standard deviation of a predetermined number of data points included in the sensor data is greater than a preset threshold, determining to perform the post-processing, and when the standard deviation of the data points is the preset threshold or smaller, determining not to perform the post-processing.

The sensor data may include a first data point corresponding to a first time point and a second data point corresponding to a second time point adjacent to the first time point, and the determining of whether to perform the post-processing may include, when a time interval between the first time point and the second time point is shorter than a preset threshold time, determining to perform the post-processing, and when the time interval is the present threshold time or longer, determining not to perform the post-processing.

The performing of the post-processing may include applying a predefined filter to a first dataset composed of a plurality of data points corresponding to a first time period to correct a value of a data point corresponding to a predetermined index among the plurality of data points, generating a second dataset corresponding to a second time period following the first time period using the data point of which the value has been corrected, and applying the predefined filter to the second dataset to correct a value of a data point corresponding to the predetermined index among a plurality of data points included in the second dataset.

The first time period may overlap at least a part of the second time period.

The applying of the predefined filter to the first dataset may include normalizing the first dataset, performing a convolutional operation on the first dataset using the predefined filter, removing distortion of the first dataset, and denormalizing the first dataset.

The normalizing of the first dataset may include subtracting a value of a data point corresponding to the last index among the plurality of data points included in the first dataset from a value of each data point included in the first dataset.

The updating of the display of the UI element may include updating the display of the UI element such that the UI element indicates a value of the postprocessed sensor data.

The method may further include calculating post-processing counts for each of a plurality of data points included in the sensor data, and the updating of the display of the UI element may include displaying a plurality of UI elements corresponding to the plurality of data points to be visually distinguishable on the basis of the post-processing counts.

The updating of the display of the UI element may include displaying the plurality of UI elements on the basis of whether the post-processing counts satisfy a predetermined count.

According to another aspect of the present disclosure, there is provided an electronic apparatus including a communication interface including at least one communication circuit, a display, a memory configured to store at least one instruction, and a processor. The processor executes the at least one instruction to perform operations of acquiring sensor data indicating an concentration of an analyte in a host, displaying a UI element indicating a value of the sensor data on the display, determining whether to perform post-processing on the sensor data on the basis of the sensor data, performing post-processing on the sensor data on the basis of a result of the determination, and updating a display of the UI element on the basis of the postprocessed sensor data.

The processor may determine to perform the post-processing when a standard deviation of a predetermined number of data points included in the sensor data is greater than a preset threshold, and may determine not to perform the post-processing when the standard deviation of the data points is the preset threshold or smaller.

The sensor data may include a first data point corresponding to a first time point and a second data point corresponding to a second time point adjacent to the first time point, and the processor may determine to perform the post-processing when a time interval between the first time point and the second time point is shorter than a preset threshold time, and may determine not to perform the post-processing when the time interval is the present threshold time or longer.

The processor may apply a predefined filter to a first dataset composed of a plurality of data points corresponding to a first time period to correct a value of a data point corresponding to a predetermined index among a plurality of data points, generate a second dataset corresponding to a second time period following the first time period using the data point of which the value has been corrected, and apply the predefined filter to the second dataset to correct a value of a data point corresponding to the predetermined index among a plurality of data points included in the second dataset.

The processor may normalize the first dataset, perform a convolutional operation on the first dataset using the predefined filter, remove distortion of the first dataset, and denormalize the first dataset.

The processor may normalize the first dataset by subtracting a value of a data point corresponding to the last index among the plurality of data points included in the first dataset from a value of each data point included in the first dataset.

The processor may update the display of the UI element such that the UI element indicates a value of the postprocessed sensor data.

The processor may calculate post-processing counts for each of a plurality of data points included in the sensor data and display a plurality of UI elements corresponding to the plurality of data points to be visually distinguishable on the basis of the post-processing counts.

The processor may display the plurality of UI elements on the basis of whether the post-processing counts satisfy a predetermined count.

Solutions to the objects of the present disclosure are not limited to those described above, and other solutions that have not been described above will be clearly understood by those of ordinary skill in the art from the present specification and the accompanying drawings.

BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:

FIG 1 is a schematic diagram of a continuous glucose monitoring system (CGMS) according to an exemplary embodiment of the present disclosure;
FIG 2 is a flowchart illustrating a method of controlling an electronic apparatus according to an exemplary embodiment of the present disclosure;
FIG 3 shows information related to sensor data according to an exemplary embodiment of the present disclosure;
FIG 4 shows an exemplary embodiment of a method of displaying sensor data (30) of FIG 3;
FIG 5 shows a dataset according to an exemplary embodiment of the present disclosure;
FIG 6 is a diagram illustrating a post-processing method according to an exemplary embodiment of the present disclosure;
FIG 7 is a diagram illustrating a post-processing method according to another exemplary embodiment of the present disclosure;
FIG 8 is a flowchart illustrating a filter application method according to an exemplary embodiment;
FIG 9 shows an exemplary embodiment of a method of displaying a first dataset (61) of FIG 6; and
FIG 10 is a block diagram of an analyte monitoring system 1000 according to an exemplary embodiment of the present disclosure.

DETAILED DESCRIPTION OF EXEMPLARY EMBODIMENTS

Terminology used in the present specification will be briefly described first, and then the present disclosure will be described in detail.

As terms used herein, general terms currently used as widely as possible will be selected in consideration of functionality in the present disclosure, but may vary depending on the intent of those of ordinary skill in the art, precedents, the advent of new technology, and the like. In particular, a term may be arbitrarily selected by the applicant. In this case, the meaning of the term will be explained in detail through the relevant description of the disclosure. Therefore, the terms used herein should be defined on the basis of their meanings and the overall content of the present disclosure rather than their names.

The present disclosure may be modified in various ways and have various embodiments, and specific embodiments will be illustrated in the drawings and described in detail. However, this does not intend to limit the present disclosure to specific embodiments, and it is to be understood that the present disclosure includes all modifications, equivalents, and substitutions within the disclosed spirit and technical scope. In describing embodiments, a detailed description of relevant known technology will be omitted when determined to obscure the subject matter of the present disclosure.

Terms such as "first," "second," and the like may be used to describe various components, but components are not limited by the terms. The terms are only used for the purpose of distinguishing one component from others.

Singular expressions include plural expressions unless the context clearly indicates otherwise. In this application, the terms "include," "have," and the like indicate the presence of features, integers, steps, operations, components, parts, or combinations thereof described in the present specification and do not preclude the presence or addition of one or more other features, integers, steps, operations, components, parts, or combinations thereof.

Hereinafter, embodiments of the present disclosure will be described in detail with reference to the accompanying drawings so that those skilled in the technical field to which the present disclosure pertains can easily implement the present disclosure. However, the present disclosure can be implemented in many different forms and is not limited to the embodiments described herein. To clearly describe the present disclosure, parts irrelevant to the description will be omitted in the drawings, and throughout the specification, like reference numerals refer to like parts.

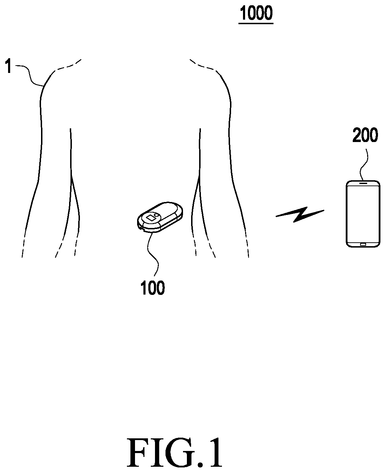

FIG 1 is a schematic diagram of a continuous glucose monitoring system (CGMS) according to an exemplary embodiment of the present disclosure.

Referring to FIG 1, an analyte monitoring system 1000 may include an analyte monitoring device 100 and an electronic apparatus 200. For example, the analyte monitoring system 1000 may be a CGMS, and the analyte monitoring device 100 may be a continuous glucose monitor (CGM). The continuous glucose monitor (CGM) may include a continuous analyte sensor. The continuous analyte sensor may be a continuous glucose sensor. The electronic apparatus 200 may be a user terminal device. As an example, the electronic apparatus 200 may be a smartphone, a tablet personal computer (PC), a smart watch, a personal digital assistant (PDA), or a dedicated receiver (e.g., a receiver).

The analyte monitoring device 100 may acquire information related to a concentration of an analyte included in a body fluid of a user 1. The analyte may include glucose and ketone. The information related to the concentrate of the analyte may include a magnitude of a signal measured by the analyte monitoring device 100. The information related to the concentration of the analyte may include a value indicating the concentration of the analyte.

The analyte monitoring device 100 may transmit the information related to the concentration of the analyte to the electronic apparatus 200 in accordance with a predetermined schedule. For example, the analyte monitoring device 100 may transmit the information related to the concentration of the analyte to the electronic apparatus 200 every five minutes.

The analyte monitoring device 100 may be attached to the body of the user 1. At least a part of the analyte monitoring device 100 may be inserted into the skin of the user 1 to be placed in the body of the user 1.

The electronic apparatus 200 may provide the information related to the concentration of the analyte to a user. The electronic apparatus 200 may display the information related to the concentration of the analyte. The information related to the concentration of the analyte provided to the user may include data of which a signal has been processed by the electronic apparatus 200. For example, the electronic apparatus 200 may generate a blood concentration value by applying a predefined algorithm to a signal detected by the analyte monitoring device 100.

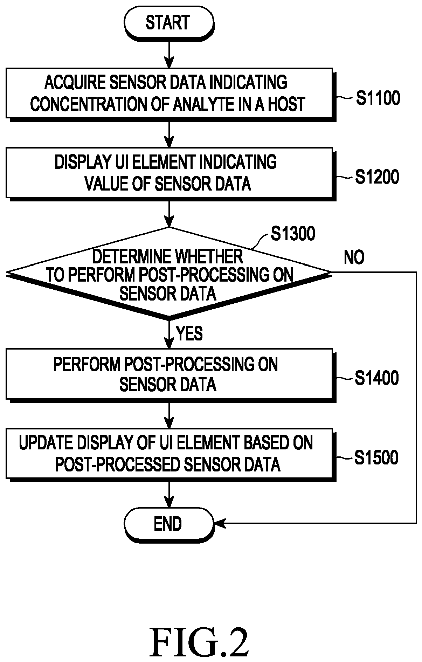

FIG 2 is a flowchart illustrating a method of controlling an electronic apparatus according to an exemplary embodiment of the present disclosure.

Referring to FIG 2, the electronic apparatus 200 may acquire sensor data that indicates an concentration of an analyte in a host (51100). The sensor data may include a plurality of data points. The plurality of data points may be time-series data having certain time intervals. A value of each data point may be an concentration value of the analyte. Meanwhile, in the present disclosure, acquiring, displaying, or processing sensor data or a data point represents acquiring, displaying, or processing a value of the sensor data or the data point.

The electronic apparatus 200 may acquire sensor data in various ways. According to an embodiment, the electronic apparatus 200 may receive a sensor signal from the analyte monitoring device 100. Then, the electronic apparatus 200 may derive sensor data by processing the sensor signal. Processing the sensor signal may include noise filtering and calibration of the sensor signal. For example, calibration of the sensor signal may be an operation of deriving an concentration value of the analyte from the sensor signal using sensitivity and/or an offset value of a sensor.

According to another embodiment, the electronic apparatus 200 may receive sensor data from the analyte monitoring device 100. In other words, the electronic apparatus 200 may receive an concentration value of the analyte from the analyte monitoring device 100. Here, the analyte monitoring device 100 may derive the sensor data by processing a sensor signal. The analyte monitoring device 100 may transmit the sensor signal and the sensor data to the electronic apparatus 200.

The electronic apparatus 200 may display user interface (UI) elements indicating values of the sensor data (S1200). For example, the electronic apparatus 200 may display values of each of the plurality of data points included in the sensor data in a two-dimensional (2D) graph. An x-axis of the graph indicates time, and a y-axis of the graph indicates blood glucose values. The electronic apparatus 200 may display a visual indicator at a position corresponding to a value and time point of each data point. The visual indicator may be a point. The visual indicator may have any of various shapes such as a circle, a polygon, an arrow, and the like.

The electronic apparatus 200 may display the latest data point differently from earlier data points. For example, the electronic apparatus 200 may display earlier data points only in a graph but display not only the latest data point in a graph but also a value of the latest data as a number. The number indicating the value of the latest data point may be displayed in a different region than a region where the graph is displayed.

The electronic apparatus 200 may determine whether to perform post-processing on the sensor data (S1300). Post-processing of the sensor data may be performed on a dataset composed of a predetermined number (e.g., ten) of data points. The electronic apparatus 200 may determine whether to perform post-processing on the dataset in accordance with whether there is an error in the dataset. When there is an error in the dataset, the electronic apparatus 200 may determine not to perform post-processing on the dataset. When there is no error in the dataset, the electronic apparatus 200 may determine to perform post-processing on the dataset.

According to an embodiment, the electronic apparatus 200 may determine whether there is an error in the dataset on the basis of a standard deviation of data points included in the dataset. Specifically, when the standard deviation is greater than a preset threshold, the electronic apparatus 200 may determine that there is no error in the dataset. On the other hand, when the standard deviation is smaller than or equal to the preset threshold, the electronic apparatus 200 may determine that there is an error in the dataset.

In other words, if the variability of the sensor data is below a preset threshold, post-processing can be omitted. By omitting post-processing, unnecessary computational tasks can be avoided, thereby saving computational resources. Additionally, since resources can be focused only on the data that requires processing, the allocation of resources can be more efficient.

According to another embodiment, the electronic apparatus 200 may determine whether there is an error in the dataset on the basis of whether there is a lost data point. When a time interval between data points constituting the dataset is a threshold time or longer due to a lost data point, a value of the dataset may be distorted in a post-processing process. If smoothing is applied to the dataset when the time intervals are irregular or greater than the threshold time, the calibrated values may differ significantly from the actual values. To prevent this, the electronic apparatus 200 may determine that there is an error in the dataset if the time interval exceeds the threshold. Although data point loss was given as an example, the same applies when the time interval between data points exceeds the threshold due to communication failures or other issues.

Specifically, the electronic apparatus 200 may determine whether there is an error (i.e., a lost data point) in the dataset on the basis of time intervals between the data points included in the dataset. For example, the dataset may include a first data point corresponding to a first time point and a second data point corresponding to a second time point adjacent to the first time point. The electronic apparatus 200 may determine whether a time interval between the first time point and the second time point is shorter than a preset threshold time. When the time interval is shorter than the preset threshold time, the electronic apparatus 200 may determine that there is no error in the dataset. When the time interval is the preset threshold time or longer, the electronic apparatus 200 may determine that there is an error in the dataset. In other words, the electronic apparatus 200 may determine there is a lost data point between the first time point and the second time point. When it is determined to perform post-processing on the sensor data (YES in S1300-), the electronic apparatus 200 may perform post-processing on the sensor data (S1400). The post-processing according to the present disclosure may include an operation that adjusts the sensor data values to make them visually easier for the user to interpret. For example, Post-processing of the sensor data may include an operation of applying a predefined filter to the sensor data to change a value of the sensor data. Post-processing of the sensor data may be performed in units of datasets composed of a predetermined number of data points. In other words, the predefined filter may be applied in units of datasets. Datasets may be composed of the latest data point and a predetermined number (e.g., nine) of earlier data points.

The predefined filter may include a smoothing filter. The smoothing filter may include a Savitzky-Golay filter.

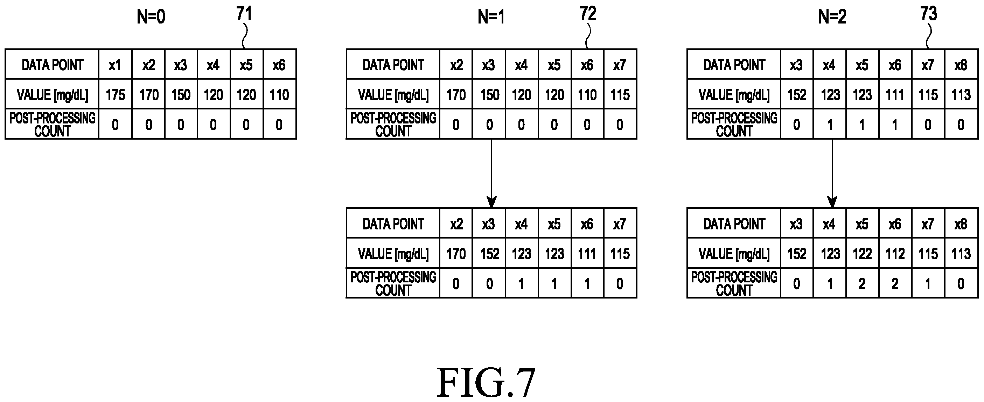

The operation of applying the predefined filter to datasets may include an operation of normalizing the datasets, an operation of performing a convolutional operation on the datasets using the predefined filter, an operation of removing distortion of the datasets, and an operation of denormalizing the datasets. Application of the filter will be described in detail below with reference to FIG 7.

When a new data point is acquired, the electronic apparatus 200 may generate a new dataset and perform post-processing on the new dataset. A time interval corresponding to the new dataset may overlap a time interval corresponding to the previously generated dataset. Accordingly, the new dataset may include some data points included in the previously generated dataset. Therefore, post-processing may be performed on one data point several times.

The value of the sensor data according to the present disclosure may be a calibrated value of the raw data measured by the sensor. In this process, the unit of the sensor data may be changed from a current value to a concentration value. The post-processing of the sensor data may involve further calibrating the initially calibrated concentration value, and this may be a retrospective calibration.

When it is determined not to perform post-processing on the sensor data (NO in S1300-), the electronic apparatus 200 may not perform post-processing on the sensor data. Therefore, the UI elements displayed by the electronic apparatus 200 may not be updated. For example, the representation of the UI elements indicating the glucose concentration value may remain unchanged.

The electronic apparatus 200 may update displays of the UI elements on the basis of the postprocessed sensor data (S1500). The electronic apparatus 200 may update the displays of the UI elements such that the UI elements indicate values of the postprocessed sensor data. For example, the electronic apparatus 200 may change a position of a visual indicator which is displayed at a first position to show a first blood glucose value before the post-processing, to a second position to show a second blood glucose value which has been updated in accordance with the post-processing. Accordingly, the user can recognize the second blood glucose value as his or her own blood glucose value.

The electronic apparatus 200 may calculate post-processing counts for each of the plurality of data points included in the sensor data. The electronic apparatus 200 may display the plurality of UI elements differently depending on a post-processing count for each data point. The electronic apparatus 200 may display a UI element indicating a value of a data point, differently depending on whether a post-processing count for the data point satisfies a predetermined count. For example, a post-processing count for the first data point may satisfy the predetermined count, and a post-processing count for the second data point may not satisfy the predetermined count.

According to an embodiment, the electronic apparatus 200 may display a first UI element corresponding to the first data point clearer or darker than a second UI element corresponding to the second data point. According to another embodiment, the electronic apparatus 200 may display a first UI element and a second UI element in different colors or patterns. According to still another embodiment, the second UI element may include a message indicating that a blood glucose value shown by the second UI element may be inaccurate. According to yet another embodiment, the electronic apparatus 200 may display a UI element corresponding to a data point on the basis of a difference value between a post-processing count for the data point and the predetermined count. For example, the UI element may be displayed lighter with an increase in the difference value.

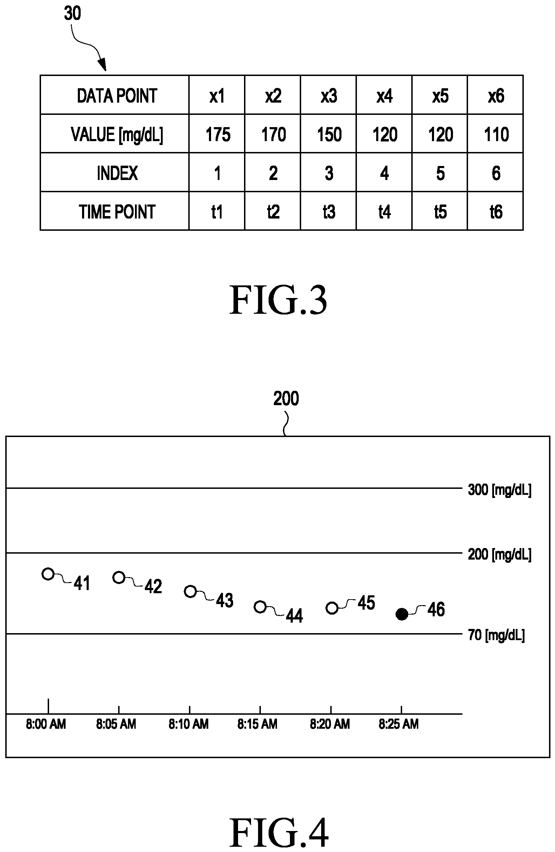

FIG 3 shows information related to sensor data according to an exemplary embodiment of the present disclosure.

Referring to FIG 3, sensor data may include data points x1, x2, x3, x4, x5, and x6. Information 30 related to the sensor data may include information related to the data points x1, x2, x3, x4, x5, and x6. Information related to the data points x1, x2, x3, x4, x5, and x6 may include values of the data points x1, x2, x3, x4, x5, and x6. The value of each data point may be a user's blood glucose value.

The information related to the data points x1, x2, x3, x4, x5, and x6 may include a sequence of the data points x1, x2, x3, x4, x5, and x6. The sequence of the data points x1, x2, x3, x4, x5, and x6 may represent the index of each data point. The index of a data point may represent a index at which the data point has been measured or derived. The order of data points may be given in ascending order over time. According to another embodiment, the order of data points may be given in descending order over time.

The information related to the data points x1, x2, x3, x4, x5, and x6 may include time points t1, t2, t3, t4, t5, and t6 corresponding to the data points x1, x2, x3, x4, x5, and x6, respectively. Here, a time point corresponding to a data point may be a time point when a value of the data point has been derived. Values of data points may not be derived at certain time intervals. For example, due to communication failure between the electronic apparatus 200 and the analyte monitoring device 100, communication of a sensor signal or a data point may be delayed. Alternatively, due to a malfunction of the electronic apparatus 200 or the analyte monitoring device 100, time points when values of data points have been actually derived may not have the certain time intervals.

FIG 4 shows an exemplary embodiment of a method of displaying sensor data 30 of FIG 3.

Referring to FIG 4, the electronic apparatus 200 may display UI elements 41, 42, 43, 44, 45, and 46 corresponding to data points x1, x2, x3, x4, x5, and x6 included in the sensor data 30. The first UI element 41 may correspond to the first data point x1, the second UI element 42 may correspond to the second data point x2, the third UI element 43 may correspond to the third data point x3, the fourth UI element 44 may correspond to the fourth data point x4, the fifth UI element 45 may correspond to the fifth data point x5, and the sixth UI element 46 may correspond to the sixth data point x6.

The electronic apparatus 200 may represent the sensor data 30 in various types of graphs. The x-axis of a graph may indicate time, and the y-axis of the graph may indicate blood glucose values. According to an embodiment, the electronic apparatus 200 may display the sensor data 30 as a point graph as shown in FIG 4. According to other embodiments, the electronic apparatus 200 may also display the sensor data 30 as a line graph, a broken line graph, or a bar graph. The line graph may be a straight line or a curved line.

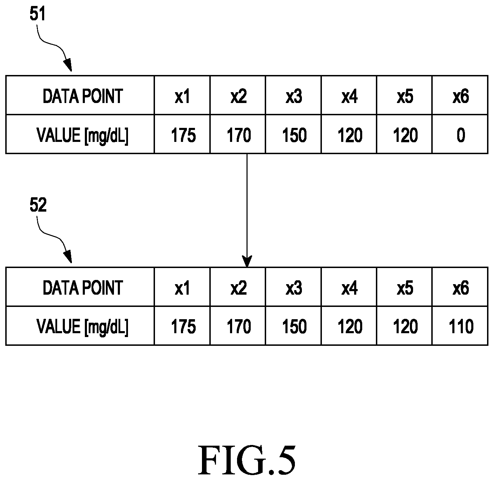

FIG 5 shows a dataset according to an exemplary embodiment of the present disclosure.

Referring to FIG 5, there is a dataset 51 composed of six data points x1, x2, x3, x4, x5, and x6. The electronic apparatus 200 may generate a dataset using a predetermined number of data points. FIG 5 shows an example in which the predetermined number is six, but the present disclosure is not limited thereto.

The electronic apparatus 200 may generate a new dataset every time a new data point is acquired. For example, the dataset 51 may be generated upon acquisition of the latest data point x6.

When the electronic apparatus 200 does not have the predetermined number of data points, the electronic apparatus 200 may not generate a dataset until there is the predetermined number of data points. For example, in the early stage of a sensor utilization period, the number of data points that the electronic apparatus 200 has may be smaller than the predetermined number. In this case, the electronic apparatus 200 may neither generate a dataset nor perform post-processing on a dataset. Therefore, a time point when a post-processing operation is performed on sensor data according to the present disclosure may be after at least a predetermined number of data points are acquired.

Meanwhile, the dataset 51 may include the data point x6 having a value of 0. For example, when application of a predefined algorithm to a sensor signal is not completed, a value of a data point may be 0. Alternatively, a value of the data point x6 may be 0 due to an internal error of the electronic apparatus 200 or any other error.

In the case of performing post-processing on the dataset 51 including the data point x6 having a value of 0, a value of the dataset 51 may be distorted in the post-processing process. Therefore, it may be necessary to correct the value of the data point x6 which is 0 into a value other than 0 or exclude the data point x6 having a value of 0 from the dataset 51 and generate the dataset 51 using a subsequent data point having a value other than 0. In the latter case, however, it is necessary to wait until a new data point having a value other than 0 is acquired, which delays generation and post-processing of the dataset 51. Accordingly, a user cannot view a value of postprocessed sensor data in real time, which is problematic. To prevent this, the electronic apparatus 200 may derive a new dataset 52 by correcting the value of the data point x6.

The electronic apparatus 200 may correct the value of the data point x6 on the basis of intensity of a sensor signal corresponding to the data point x6. Specifically, the electronic apparatus 200 may derive a new value of the data point x6 by applying the predefined algorithm to the sensor signal corresponding to the data point x6. For example, an operation of applying the predefined algorithm may include an operation of deriving a blood glucose value from the sensor signal using sensor sensitivity and/or an offset value.

With such an operation in which the electronic apparatus 200 corrects the dataset 51, it is possible to prevent a value of the dataset 51 from being distorted in the post-processing process. Also, it is unnecessary to wait for a new subsequent data, and a user can view a value of postprocessed data in real time.

When the dataset 52 is generated, the electronic apparatus 200 may determine whether to perform post-processing on the dataset 52. As described above, the electronic apparatus 200 may determine whether to perform post-processing on the basis of whether there is an error in the dataset 52.

According to an embodiment, the electronic apparatus 200 may determine whether there is an error in the dataset 52 on the basis of a standard deviation of the data points x1, x2, x3, x4, x5, and x6. Specifically, when the standard deviation of the data points x1, x2, x3, x4, x5, and x6 is greater than a preset threshold, the electronic apparatus 200 may determine that there is no error in the dataset 52. On the other hand, when the standard deviation of the data points x1, x2, x3, x4, x5, and x6 is smaller than or equal to the preset threshold, the electronic apparatus 200 may determine that there is an error in the dataset 52.

According to another embodiment, the electronic apparatus 200 may determine whether there is an error in the dataset 52 on the basis of time intervals between adjacent data points among the data points x1, x2, x3, x4, x5, and x6. Referring back to FIG 3, the electronic apparatus 200 may calculate an interval between the first time point t1 and the second time point t2, an interval between the second time point t2 and the third time point t3, an interval between the third time point t3 and the fourth time point t4, an interval between the fourth time point t4 and the fifth time point t5, and an interval between the fifth time point t5 and the sixth time point t6. When all the calculated intervals are shorter than a preset threshold time, the electronic apparatus 200 may determine that there is no error in the dataset 52. On the other hand, when any one of the calculated intervals is longer than or equal to the preset threshold time, the electronic apparatus 200 may determine that there is an error in the dataset 52.

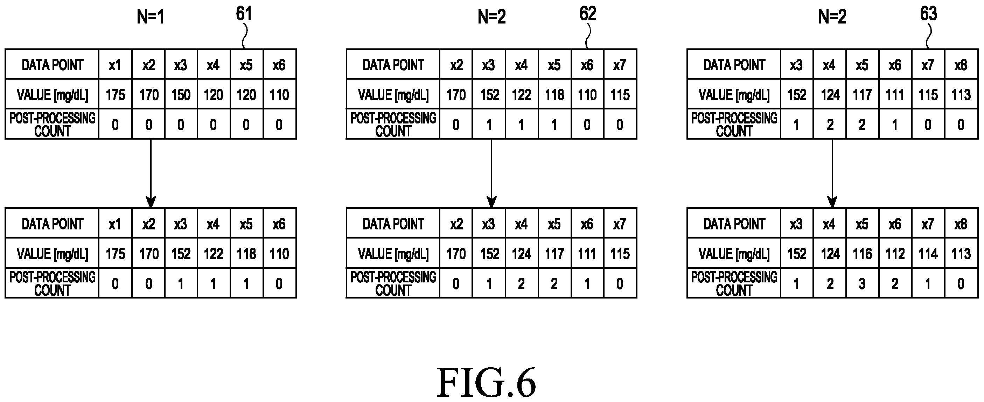

FIG 6 is a diagram illustrating a post-processing method according to an exemplary embodiment of the present disclosure.

Referring to FIG 6, the electronic apparatus 200 may perform post-processing on a first dataset 61. For example, the electronic apparatus 200 may apply a predefined filter to the first dataset 61. Accordingly, values of some of the data points x1, x2, x3, x4, x5, and x6 corresponding to predetermined indexes may be updated.

Even when the filter is applied to all the data points x1, x2, x3, x4, x5, and x6, only values of the data points x3, x4, and x5 corresponding to the predetermined indexes may be updated, and values of the other data points x1, x2, and x6 may not be updated. The values of the data points x1 and x2 corresponding to first some indexes may be distorted due to filter application. Therefore, the values of the data points x1 and x2 may not be updated with values that are acquired after the filter is applied. Since the value of the data point x6 corresponding to the last index is the most recent blood glucose value that the user is most interested in, it may be confusing to the user when the value changes in post-processing. Therefore, the value of the data point x6 may not be updated with a value that is acquired after the filter is applied.

After post-processing is performed on the dataset 61, the electronic apparatus 200 may acquire post-processing counts for each of the data points x1, x2, x3, x4, x5, and x6. Specifically, the post-processing counts for the data points x3, x4, and x5 corresponding to the predetermined indexes are increased, while the post-processing counts for the other data points x1, x2, and x6 are not increased. Therefore, a post-processing count N of the dataset 61 may differ from a post-processing count for each individual data point. Also, some data points may have different post-processing counts.

When a new data point x7 is acquired, the electronic apparatus 200 may acquire a second dataset 62. The second dataset 62 may be composed of a part (x2, x3, x4, x5, and x6) of the postprocessed first dataset 61 and the new data point x7. In other words, time periods of adjacent datasets may overlap, and the adjacent datasets may include data points in common.

The electronic apparatus 200 may perform post-processing on the second dataset 62. Specifically, the electronic apparatus 200 may apply the predefined filter to the second dataset 62. Accordingly, values of the data points x4, x5, and x6 corresponding to the predetermined indexes may be updated among the data points x2, x3, x4, x5, x6, and x7. Post-processing counts for the data points x4, x5, and x6 corresponding to the predetermined indexes may be increased by 1.

When a new data point x8 is acquired, the electronic apparatus 200 may acquire a third dataset 63. The third dataset 63 may be composed of a part (x3, x4, x5, x6, and x7) of the postprocessed second dataset 62 and the new data point x8.

The electronic apparatus 200 may perform post-processing on the third dataset 63. Specifically, the electronic apparatus 200 may apply the predefined filter to the third dataset 63. Accordingly, values of the data points x5, x6, and 7 corresponding to the predetermined indexes may be updated among the data points x3, x4, x5, x6, x7, and x8. Post-processing counts for the data points x5, x6, and x7 corresponding to the predetermined indexes may be increased by 1.

As described above, every time a new data point is acquired, the electronic apparatus 200 may generate a dataset, determine whether to perform post-processing on the dataset, and then perform post-processing when it is determined to perform post-processing. While repeating post-processing on several datasets, the electronic apparatus 200 may store post-processing counts for individual data points.

FIG 7 is a diagram illustrating a post-processing method according to another exemplary embodiment of the present disclosure.

Referring to FIG 7, the electronic apparatus 200 may generate a first dataset 71. The electronic apparatus may determine whether there is an error in the first dataset 71 to determine whether to perform post-processing on the first dataset 71. For example, a time interval between a first time point corresponding to a first data point x1 and a second time point corresponding to a second data point x2 may be longer than a preset threshold time (e.g., 10 minutes). In this case, the electronic apparatus 200 may determine that there is an error in the first dataset 71 and may not perform post-processing on the first dataset 71.

There may be no error in either of a second dataset 72 and a third dataset 73. Therefore, the electronic apparatus 200 may perform post-processing on the second dataset 72 and the third dataset 73 in the same way as described in FIG 6 and acquire a post-processing count for each data point. Comparing FIGS. 6 and 7, each of the data points x3, x4, and x5 varies in the post-processing count. This is because post-processing is not applied to the first dataset 71 due to the error. In this way, a post-processing count for each data point may vary depending on whether there is an error.

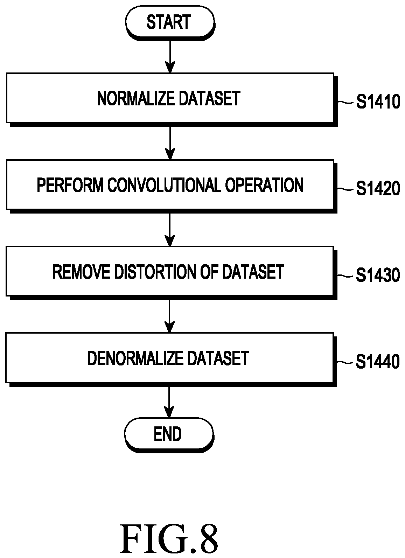

FIG 8 is a flowchart illustrating a filter application method according to an exemplary embodiment.

Referring to FIG 8, the electronic apparatus 200 may normalize a dataset (S1410). The electronic apparatus 200 may normalize values of each of a plurality of data points included in the dataset.

According to an embodiment, the electronic apparatus 200 may subtract a value of a data point corresponding to the last index among the plurality of data points from a value of each data point as shown in Equation 1. Then, the electronic apparatus 200 may divide a value obtained by subtracting the value of the data point corresponding to the last index from the value of each data point by a standard deviation of the plurality of data points.

[Equation 1]

$$x_{norm} = \frac{x - x(end)}{std(X)}$$

In Equation 1, x is a value of a data point before normalization, x(end) is a value of a data point corresponding to the last index of a dataset X, std(X) is a standard deviation of values of data points included in the dataset X, and $x_{norm}$ is a normalized value of the data point. Equation 1 may be referred to as "zero-end normalization" or "zero-end norm."

When normalization is performed using the last data point of each dataset, a difference between a postprocessed data point and the latest data point which has not been postprocessed can be minimized. Accordingly, data points around the latest data point can be smoothed.

According to another embodiment, the electronic apparatus 200 may subtract an average of the plurality of data points from a value of each data point. Then, the electronic apparatus 200 may divide a value obtained by subtracting the average from the value of each data point by the standard deviation of the plurality of data points.

The electronic apparatus 200 may perform a convolutional operation on the dataset (S1420). The electronic apparatus 200 may perform the convolutional operation using a predefined smoothing filter.

The electronic apparatus 200 may remove distortion of the dataset (S1430). The electronic apparatus 200 may remove a part of a result of the convolutional operation corresponding to first some indexes. The reason is that the part corresponding to first some indexes may include distortion due to filter characteristics. This is related to FIG 6 in which values of the data points x2 and x3 included in the second dataset 62 are not updated. The reason is that, in a process of applying the filter to the second dataset 62, the values of the data points x2 and x3 corresponding to first some indexes may be distorted. The electronic apparatus 200 does not update the values of the data points x2 and x3 to prevent the data points x2 and x3 from being distorted by post-processing.

Meanwhile, a range of removed indexes may be determined on the basis of the length of a filter coefficient. For example, the number of removed indexes may be half the length of the filter coefficient. When the length of the filter coefficient is an odd number, the number of removed indexes may be half the length of the filter coefficient with decimal places discarded.

The electronic apparatus 200 may denormalize the dataset (S 1440). The electronic apparatus 200 may denormalize the values of the plurality of data points included in the dataset. The electronic apparatus 200 may update the values of the data points having predetermined indexes on the basis of the denormalized values.

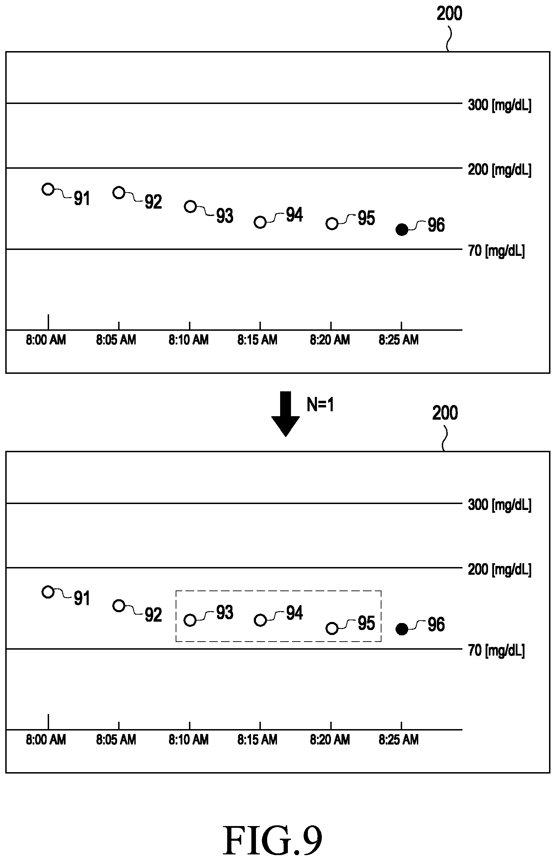

FIG 9 shows an exemplary embodiment of a method of displaying the first dataset 61 of FIG 6.

Referring to FIG 9, the electronic apparatus 200 may display UI elements 91, 92, 93, 94, 95, and 96 corresponding to the data points x1, x2, x3, x4, x5, and x6 included in the first dataset 61. The UI element 96 corresponding to the latest data point x6 may be displayed to be visually distinguishable from the other UI elements 91, 92, 93, 94, and 95.

As described above, the electronic apparatus 200 performs post-processing on the first dataset 61, and values of the data points x3, x4, and x5 may be updated accordingly. The electronic apparatus 200 may update displays of the UI elements 93, 94, and 95 on the basis of the updated values. As shown in the drawing, the electronic apparatus 200 may change positions of the UI elements 93, 94, and 95 to show the updated values.

Every time a new data point is acquired, the electronic apparatus 200 may generate a new dataset and perform post-processing on the new dataset. The generated new dataset may include some of data points included in a dataset corresponding to an immediately preceding time period. Accordingly, post-processing may be performed on each data point several times, and a display of a UI element corresponding to each data point may be updated several times. When there is no error in a dataset, a post-processing count for one data point may be the same as the number of data points of which values are updated by single processing.

Although not shown in the drawing, the electronic apparatus 200 may update a display of a UI element corresponding to each data point on the basis of a post-processing count for the data point. For example, the electronic apparatus 200 may differently display a data point satisfying a predetermined post-processing count and a data point not satisfying the predetermined post-processing count. Specifically, UI elements corresponding to the data points may vary in shape or color.

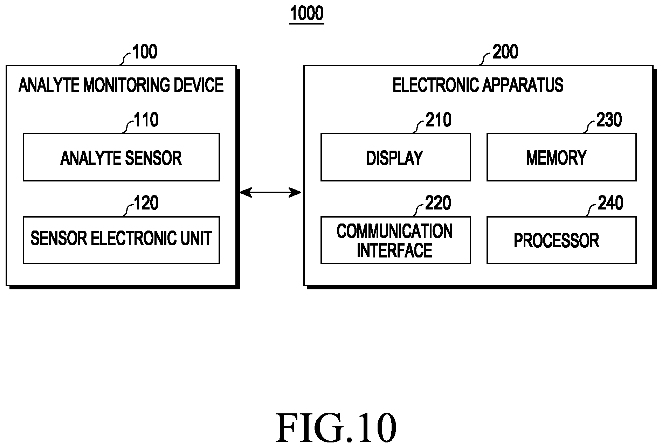

FIG 10 is a block diagram of an analyte monitoring system 1000 according to an exemplary embodiment of the present disclosure.

Referring to FIG 10, the analyte monitoring system 1000 may include an analyte monitoring device 100 and an electronic apparatus 200.

The analyte monitoring device 100 may include an analyte sensor 110 and a sensor electronic unit 120.

The analyte sensor 110 may be an element for sensing an analyte signal (or sensor signal). The analyte sensor 110 may include a sensor probe of which at least a part is inserted into the body. In the sensor probe, a sensing region reacting to glucose in a body may be formed to measure a concentration of glucose in a host. The analyte sensor 110 may be a continuous glucose sensor.

The sensor electronic unit 120 may include at least one communication interface. The sensor electronic unit 120 may communicate with the electronic apparatus 200 via the communication interface. For example, the communication interface may include a Bluetooth module, a Bluetooth Low Energy (BLE) module, a radio frequency (RF) module, and a near field communication (NFC) module.

The sensor electronic unit 120 may transmit the sensor signal acquired through the analyte sensor 110 to the electronic apparatus 200. Also, the sensor electronic unit 120 may transmit a value of a data point derived from the sensor signal to the electronic apparatus 200. The sensor electronic unit 120 may transmit a sensor signal or a value of a data point to the electronic apparatus 200 at predetermined intervals (e.g., five minutes).

The sensor electronic unit 120 may include a memory that stores an operating system (OS) for controlling overall operations of the components of the analyte monitoring device 100 and instructions or data related to the components of the analyte monitoring device 100. Also, the sensor electronic unit 120 may include a processor that is electrically connected to the memory to control overall functions and operations of the analyte monitoring device 100.

The electronic apparatus 200 may include a display 210, a communication interface 220, a memory 230, and a processor 240. The electronic apparatus 200 may be a user terminal device such as a smartphone.

The display 210 may output sensor data. For example, the display 210 may output a blood glucose value of a user.

The communication interface 220 may include at least one communication circuit. The communication interface 220 may receive the sensor signal from the analyte monitoring device 100. The communication interface 220 may receive a value of a data point from the analyte monitoring device 100.

The memory 230 may store an OS for controlling overall operations of the components of the electronic apparatus 200 and instructions or data related to the components of the electronic apparatus 200. The memory 230 may be implemented as a non-volatile memory (e.g., a hard disk, a solid state drive (SSD), or a flash memory), a volatile memory, or the like.

The processor 240 may be electrically connected to the memory 230 to control overall functions and operations of the electronic apparatus 200. The processor 240 may control the electronic apparatus 200 by executing instructions stored in the memory 230.

The processor 240 may acquire sensor data indicating an concentration of an analyte in a host. According to an embodiment, the processor 240 may derive the sensor data from the sensor signal received from the analyte monitoring device 100 through the communication interface 220. According to another embodiment, the processor 240 may receive sensor data indicating an concentration of an analyte from the analyte monitoring device 100 through the communication interface 220.

The processor 240 may display a UI element indicating a value of the sensor data on the display 210. The processor 240 may control the display such that the UI element is displayed.

The processor 240 may determine whether to perform post-processing on the sensor data on the basis of the sensor data. According to an embodiment, when a standard deviation of a predetermined number of data points included in the sensor data is greater than a preset threshold, the processor 240 may determine to perform post-processing. On the other hand, when the standard deviation is smaller than or equal to the preset threshold, the processor 240 may determine not to perform post-processing.

According to another embodiment, the processor 240 may determine whether to perform post-processing on the basis of time intervals between adjacent data points. For example, the processor 240 may acquire a first data point corresponding to a first time point and a second data point corresponding to a second time point adjacent to the first data point. When a time interval between the first time point and the second time point is shorter than a preset threshold time, the processor 240 may determine to perform post-processing. On the other hand, when the time interval is the preset threshold time or longer, the processor 240 may determine not to perform post-processing.

The processor 240 may perform post-processing on the sensor data. The processor 240 may correct a value of a data point corresponding to a predetermined index among a plurality of data points corresponding to the first time period by applying a predefined filter to a first dataset composed of the plurality of data points. The processor 240 may generate a second dataset corresponding to the second time period following the first time period using the data point of which the value has been corrected. The processor 240 may correct a value of a data point corresponding to the predetermined index among a plurality of data points included the second dataset by applying the predefined filter to the second dataset. Here, the first time period may overlap at least a part of the second time period.

The processor 240 may apply the predefined filter to a dataset. The processor 240 may normalize the first dataset. For example, the processor 240 may normalize the first dataset by subtracting a value of a data point corresponding to the last index among the plurality of data points included in the first dataset from a value of each data point included in the first dataset.

The processor 240 may perform a convolutional operation on the first dataset using the predefined filter. The processor 240 may remove distortion of the first dataset. The processor 240 may denormalize the first dataset.

The processor 240 may update a display of a UI element on the basis of the postprocessed sensor data. For example, the processor 240 may change the display of the UI element such that the UI element shows a value of the postprocessed sensor data.

The processor 240 may acquire post-processing counts for each of the plurality of data points included in the sensor data. The processor 240 may display a plurality of UI elements corresponding to the plurality of data points to be visually distinguishable on the basis of the post-processing counts. For example, the processor 240 may display the plurality of UI elements on the basis of whether the post-processing counts satisfy a predetermined count.

According to an embodiment of the present disclosure, it is possible to minimize distortion of a blood glucose concentration value provided to a user.

According to an embodiment of the present disclosure, a user can conveniently view blood glucose concentrations. Accordingly, convenience and satisfaction of the user can be improved.

Other effects that can be achieved or expected from embodiments of the present disclosure have been disclosed explicitly or implicitly in the detailed description of the embodiments of the present disclosure. For example, various effects expected according to embodiments of the present disclosure have been disclosed in the above description.

Other aspects, advantages, and features of the present disclosure will become apparent to those of ordinary skill in the art from the above description, which discloses various embodiments of the present invention with reference to the accompanying drawings.

The variety of embodiments described above can be implemented in a recording medium that can be read by a computer or a similar device using software, hardware, or a combination thereof. In some cases, the embodiments described herein may be implemented as a processor. When the embodiments are implemented as software, embodiments of procedures, functions, and the like described herein may be implemented as separate software modules. Each of the software modules may perform one or more functions and operations described herein.

Computer instructions for performing processing operations according to the variety of embodiments of the present disclosure described above may be stored in a non-transitory computer-readable recording medium. These computer instructions stored in the non-transitory computer-readable recording medium may cause a specific device to perform a processing operation according to the variety of embodiments described above when executed by a processor.

The non-transitory computer-readable medium is not a medium that stores data for a short moment, such as a register, a cache, a memory, or the like, but a machine-readable medium that stores data semi-permanently. Examples of the non-transitory computer-readable medium may include a compact disc (CD), a digital versatile disc (DVD), a hard disk, a Blu-ray disc, a Universal Serial Bus (USB) memory, a memory card, a read-only memory (ROM), and the like.

The machine-readable medium may be provided in the form of a non-transitory storage medium. Here, the "non-transitory storage medium" is a tangible device that does not include any signal (e.g., electromagnetic waves), and the term is used regardless of whether data is stored in a storage medium semi-permanently or temporarily. For example, the "non-transitory storage medium" may include a buffer in which data is temporarily stored.

Methods according to various embodiments disclosed in the present document may be included and provided in a computer program product. The computer program product may be traded as a product between a seller and a purchaser. The computer program product may be distributed in the form of a machine-readable storage medium (e.g., a CD-ROM) or distributed (e.g., downloaded or uploaded) online via an application store (e.g., PlayStore™) or between two user devices (e.g., smart phones) directly. In the case of online distribution, at least a part of the computer program product (e.g., a downloadable app) may be temporarily generated or at least temporarily stored in a machine-readable storage medium such as memory of a manufacturer's server a server of the application store, or a relay server.

Although exemplary embodiments of the present disclosure have been illustrated and described above, the present disclosure is not limited thereto. Those of ordinary skill in the art can make various modified embodiments without departing from the gist of the present disclosure described in the claims, and it is to be understood that these modified embodiments fall within the technical spirit or scope of the present disclosure.

## Claims

1. A method of controlling an electronic apparatus, the method comprising:

   acquiring sensor data indicating an concentration of an analyte in a host;
   displaying a user interface (UI) element indicating a value of the sensor data;
   determining whether to perform post-processing on the sensor data on the basis of the sensor data;
   performing post-processing on the sensor data on the basis of a result of the determination; and
   updating a display of the UI element on the basis of the postprocessed sensor data.

2. The method of claim 1, wherein the determining of whether to perform the post-processing comprises:

   when a standard deviation of a predetermined number of data points included in the sensor data is greater than a preset threshold, determining to perform the post-processing; and
   when the standard deviation of the data points is the preset threshold or smaller, determining not to perform the post-processing.

3. The method of claim 1, wherein the sensor data includes a first data point corresponding to a first time point and a second data point corresponding to a second time point adjacent to the first time point, and
   the determining of whether to perform the post-processing comprises:

when a time interval between the first time point and the second time point is shorter than a preset threshold time, determining to perform the post-processing; and

when the time interval is the present threshold time or longer, determining not to perform the post-processing.

4. The method of claim 1, wherein the performing of the post-processing comprises:

applying a predefined filter to a first dataset composed of a plurality of data points corresponding to a first time period to correct a value of a data point corresponding to a predetermined index among the plurality of data points;

generating a second dataset corresponding to a second time period following the first time period using the data point of which the value has been corrected; and

applying the predefined filter to the second dataset to correct a value of a data point corresponding to the predetermined index among a plurality of data points included in the second dataset.

5. The method of claim 4, wherein the first time period overlaps at least a part of the second time period.

6. The method of claim 4, wherein the applying of the predefined filter to the first dataset comprises:

normalizing the first dataset;

performing a convolutional operation on the first dataset using the predefined filter;

removing distortion of the first dataset; and

denormalizing the first dataset.

7. The method of claim 6, wherein the normalizing of the first dataset comprises subtracting a value of a data point corresponding to a last index among the plurality of data points included in the first dataset from a value of each data point included in the first dataset.

8. The method of claim 1, wherein the updating of the display of the UI element comprises updating the display of the UI element such that the UI element indicates a value of the postprocessed sensor data.

9. The method of claim 1, further comprising calculating post-processing counts for each of a plurality of data points included in the sensor data,

wherein the updating of the display of the UI element comprises displaying a plurality of UI elements corresponding to the plurality of data points to be visually distinguishable on the basis of the post-processing counts.

10. The method of claim 9, wherein the updating of the display of the UI element comprises displaying the plurality of UI elements on the basis of whether the post-processing counts satisfy a predetermined count.

11. An electronic apparatus comprising:

a communication interface including at least one communication circuit;

a display;

a memory configured to store at least one instruction; and

a processor,

wherein the processor executes the at least one instruction to perform operations of

acquiring sensor data indicating an concentration of an analyte in a host;

displaying a user interface (UI) element indicating a value of the sensor data on the display;

determining whether to perform post-processing on the sensor data on the basis of the sensor data;

performing post-processing on the sensor data on the basis of a result of the determination; and

updating a display of the UI element on the basis of the postprocessed sensor data.

12. The electronic apparatus of claim 11, wherein the processor determines to perform the post-processing when a standard deviation of a predetermined number of data points included in the sensor data is greater than a preset threshold, and determines not to perform the post-processing when the standard deviation of the data points is the preset threshold or smaller.

13. The electronic apparatus of claim 11, wherein the sensor data includes a first data point corresponding to a first time point and a second data point corresponding to a second time point adjacent to the first time point, and

the processor determines to perform the post-processing when a time interval between the first time point and the second time point is shorter than a preset threshold time, and determines not to perform the post-processing when the time interval is the present threshold time or longer.

14. The electronic apparatus of claim 11, wherein the processor applies a predefined filter to a first dataset composed of a plurality of data points corresponding to a first time period to correct a value of a data point corresponding to a predetermined index among a plurality of data points,

generate a second dataset corresponding to a second time period following the first time period using the data point of which the value has been corrected, and

apply the predefined filter to the second dataset to correct a value of a data point corresponding to the predetermined index among a plurality of data points included in the second dataset.

15. The electronic apparatus of claim 14, wherein the first time period overlaps at least a part of the second time period.

16. The electronic apparatus of claim 14, wherein the processor normalizes the first dataset, performs a convolutional operation on the first dataset using the predefined filter, removes distortion of the first dataset, and denormalizes the first dataset.

17. The electronic apparatus of claim 16, wherein the processor normalizes the first dataset by subtracting a value of a data point corresponding to a last index among the plurality of data points included in the first dataset from a value of each data point included in the first dataset.

18. The electronic apparatus of claim 11, wherein the processor updates the display of the UI element such that the UI element indicates a value of the postprocessed sensor data.

19. The electronic apparatus of claim 11, wherein the processor calculates post-processing counts for each of a plurality of data points included in the sensor data and displays a plurality of UI elements corresponding to the plurality of data points to be visually distinguishable on the basis of the post-processing counts.

20. The electronic apparatus of claim 19, wherein the processor displays the plurality of UI elements on the basis of whether the post-processing counts satisfy a predetermined count.

1000

1

200

100

# FIG.1

START

ACQUIRE SENSOR DATA INDICATING CONCENTRATION OF ANALYTE IN A HOST — S1100

DISPLAY UI ELEMENT INDICATING VALUE OF SENSOR DATA — S1200

S1300
DETERMINE WHETHER TO PERFORM POST-PROCESSING ON SENSOR DATA

NO

YES

PERFORM POST-PROCESSING ON SENSOR DATA — S1400

UPDATE DISPLAY OF UI ELEMENT BASED ON POST-PROCESSED SENSOR DATA — S1500

END

# FIG.2

30

| DATA POINT | x1 | x2 | x3 | x4 | x5 | x6 |
|---|---|---|---|---|---|---|
| VALUE [mg/dL] | 175 | 170 | 150 | 120 | 120 | 110 |
| INDEX | 1 | 2 | 3 | 4 | 5 | 6 |
| TIME POINT | t1 | t2 | t3 | t4 | t5 | t6 |

# FIG.3

200

300 [mg/dL]

200 [mg/dL]

○ 41    ○ 42    ○ 43    ○ 44    ○ 45    ● 46

70 [mg/dL]

8:00 AM    8:05 AM    8:10 AM    8:15 AM    8:20 AM    8:25 AM

# FIG.4

51

| DATA POINT | x1 | x2 | x3 | x4 | x5 | x6 |
|---|---|---|---|---|---|---|
| VALUE [mg/dL] | 175 | 170 | 150 | 120 | 120 | 0 |

52

| DATA POINT | x1 | x2 | x3 | x4 | x5 | x6 |
|---|---|---|---|---|---|---|
| VALUE [mg/dL] | 175 | 170 | 150 | 120 | 120 | 110 |

# FIG.5

N=1  61

| DATA POINT | x1 | x2 | x3 | x4 | x5 | x6 |
|---|---|---|---|---|---|---|
| VALUE [mg/dL] | 175 | 170 | 150 | 120 | 120 | 110 |
| POST-PROCESSING COUNT | 0 | 0 | 0 | 0 | 0 | 0 |

↓

| DATA POINT | x1 | x2 | x3 | x4 | x5 | x6 |
|---|---|---|---|---|---|---|
| VALUE [mg/dL] | 175 | 170 | 152 | 122 | 118 | 110 |
| POST-PROCESSING COUNT | 0 | 0 | 1 | 1 | 1 | 0 |

N=2  62

| DATA POINT | x2 | x3 | x4 | x5 | x6 | x7 |
|---|---|---|---|---|---|---|
| VALUE [mg/dL] | 170 | 152 | 122 | 118 | 110 | 115 |
| POST-PROCESSING COUNT | 0 | 1 | 1 | 1 | 0 | 0 |

↓

| DATA POINT | x2 | x3 | x4 | x5 | x6 | x7 |
|---|---|---|---|---|---|---|
| VALUE [mg/dL] | 170 | 152 | 124 | 117 | 111 | 115 |
| POST-PROCESSING COUNT | 0 | 1 | 2 | 2 | 1 | 0 |

N=2  63

| DATA POINT | x3 | x4 | x5 | x6 | x7 | x8 |
|---|---|---|---|---|---|---|
| VALUE [mg/dL] | 152 | 124 | 117 | 111 | 115 | 113 |
| POST-PROCESSING COUNT | 1 | 2 | 2 | 1 | 0 | 0 |

↓

| DATA POINT | x3 | x4 | x5 | x6 | x7 | x8 |
|---|---|---|---|---|---|---|
| VALUE [mg/dL] | 152 | 124 | 116 | 112 | 114 | 113 |
| POST-PROCESSING COUNT | 1 | 2 | 3 | 2 | 1 | 0 |

FIG.6

EP 4 553 846 A1

EP 4 553 846 A1

N=0    71

| DATA POINT | x1 | x2 | x3 | x4 | x5 | x6 |
|---|---|---|---|---|---|---|
| VALUE [mg/dL] | 175 | 170 | 150 | 120 | 120 | 110 |
| POST-PROCESSING COUNT | 0 | 0 | 0 | 0 | 0 | 0 |

N=1    72

| DATA POINT | x2 | x3 | x4 | x5 | x6 | x7 |
|---|---|---|---|---|---|---|
| VALUE [mg/dL] | 170 | 150 | 120 | 120 | 110 | 115 |
| POST-PROCESSING COUNT | 0 | 0 | 0 | 0 | 0 | 0 |

| DATA POINT | x2 | x3 | x4 | x5 | x6 | x7 |
|---|---|---|---|---|---|---|
| VALUE [mg/dL] | 170 | 152 | 123 | 123 | 111 | 115 |
| POST-PROCESSING COUNT | 0 | 0 | 1 | 1 | 1 | 0 |

N=2    73

| DATA POINT | x3 | x4 | x5 | x6 | x7 | x8 |
|---|---|---|---|---|---|---|
| VALUE [mg/dL] | 152 | 123 | 123 | 111 | 115 | 113 |
| POST-PROCESSING COUNT | 0 | 1 | 1 | 1 | 0 | 0 |

| DATA POINT | x3 | x4 | x5 | x6 | x7 | x8 |
|---|---|---|---|---|---|---|
| VALUE [mg/dL] | 152 | 123 | 122 | 112 | 115 | 113 |
| POST-PROCESSING COUNT | 0 | 1 | 2 | 2 | 1 | 0 |

FIG.7

START

NORMALIZE DATASET ~ S1410

PERFORM CONVOLUTIONAL OPERATION ~ S1420

REMOVE DISTORTION OF DATASET ~ S1430

DENORMALIZE DATASET ~ S1440

END

# FIG.8

FIG.9

1000

```
┌─────────────────────────────┐        ┌─────────────────────────────────────────────────┐
│           ⌐100              │        │                    ⌐200                          │
│  ANALYTE MONITORING DEVICE  │        │              ELECTRONIC APPARATUS                │
│                             │        │                                                 │
│         ⌐110                │        │       ⌐210                  ⌐230                │
│   ┌───────────────────┐     │        │  ┌──────────────┐     ┌──────────────┐          │
│   │  ANALYTE SENSOR   │     │◄──────►│  │   DISPLAY    │     │    MEMORY    │          │
│   └───────────────────┘     │        │  └──────────────┘     └──────────────┘          │
│         ⌐120                │        │       ⌐220                  ⌐240                │
│   ┌───────────────────┐     │        │  ┌──────────────┐     ┌──────────────┐          │
│   │ SENSOR ELECTRONIC │     │        │  │COMMUNICATION │     │  PROCESSOR   │          │
│   │       UNIT        │     │        │  │  INTERFACE   │     │              │          │
│   └───────────────────┘     │        │  └──────────────┘     └──────────────┘          │
└─────────────────────────────┘        └─────────────────────────────────────────────────┘
```

# FIG.10

Europäisches
Patentamt

European
Patent Office

Office européen
des brevets

# EUROPEAN SEARCH REPORT

Application Number

EP 24 20 8579

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/289823 A1 (RACK-GOMER ANNA LEIGH [US] ET AL) 15 October 2015 (2015-10-15) * paragraphs [0003], [13ff], [0144]; figures 2, 3, 4 * | 1-20 | INV. G16H40/63 G16H40/67 |
| A | US 2023/140651 A1 (GARCIA ARTURO [US] ET AL) 4 May 2023 (2023-05-04) * paragraph [0207] * | 2,12 | |
| A | US 2006/017923 A1 (RUCHTI TIMOTHY L [US] ET AL) 26 January 2006 (2006-01-26) * the whole document * | 1-20 | |

TECHNICAL FIELDS
SEARCHED     (IPC)

G16H

The present search report has been drawn up for all claims

| Place of search | Date of completion of the search | Examiner |
|---|---|---|
| Munich | 25 March 2025 | Laub, Christoph |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

## ANNEX TO THE EUROPEAN SEARCH REPORT
## ON EUROPEAN PATENT APPLICATION NO.

EP 24 20 8579

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2025

| Patent document cited in search report | Publication date | Patent family member(s) | | Publication date |
|---|---|---|---|---|
| US 2015289823 A1 | 15-10-2015 | AU | 2015244291 A1 | 04-08-2016 |
| | | AU | 2017225153 A1 | 05-10-2017 |
| | | AU | 2019200136 A1 | 31-01-2019 |
| | | AU | 2021202444 A1 | 13-05-2021 |
| | | AU | 2022287585 A1 | 02-02-2023 |
| | | AU | 2024216474 A1 | 19-09-2024 |
| | | CA | 2936774 A1 | 15-10-2015 |
| | | CA | 3223238 A1 | 15-10-2015 |
| | | CN | 106415556 A | 15-02-2017 |
| | | CN | 111968737 A | 20-11-2020 |
| | | EP | 3128913 A1 | 15-02-2017 |
| | | EP | 3434184 A1 | 30-01-2019 |
| | | EP | 3437558 A1 | 06-02-2019 |
| | | EP | 4503047 A2 | 05-02-2025 |
| | | JP | 7043531 B2 | 29-03-2022 |
| | | JP | 7297964 B2 | 26-06-2023 |
| | | JP | 2017515520 A | 15-06-2017 |
| | | JP | 2020078648 A | 28-05-2020 |
| | | JP | 2022079506 A | 26-05-2022 |
| | | JP | 2023134431 A | 27-09-2023 |
| | | US | 2015289821 A1 | 15-10-2015 |
| | | US | 2015289823 A1 | 15-10-2015 |
| | | US | 2024282426 A1 | 22-08-2024 |
| | | WO | 2015156965 A1 | 15-10-2015 |
| US 2023140651 A1 | 04-05-2023 | AU | 2016321249 A1 | 22-02-2018 |
| | | AU | 2020202553 A1 | 07-05-2020 |
| | | AU | 2022200905 A1 | 03-03-2022 |
| | | AU | 2024201184 A1 | 14-03-2024 |
| | | CA | 2990843 A1 | 16-03-2017 |
| | | CN | 107771056 A | 06-03-2018 |
| | | CN | 113974619 A | 28-01-2022 |
| | | EP | 3346906 A1 | 18-07-2018 |
| | | EP | 4461218 A2 | 13-11-2024 |
| | | JP | 7488023 B2 | 21-05-2024 |
| | | JP | 7571101 B2 | 22-10-2024 |
| | | JP | 2018532440 A | 08-11-2018 |
| | | JP | 2022188264 A | 20-12-2022 |
| | | JP | 2025004084 A | 14-01-2025 |
| | | US | 2023140651 A1 | 04-05-2023 |
| | | US | 2024180425 A1 | 06-06-2024 |
| | | WO | 2017044654 A1 | 16-03-2017 |
| US 2006017923 A1 | 26-01-2006 | US | 2006017923 A1 | 26-01-2006 |
| | | WO | 2006017700 A2 | 16-02-2006 |

EPO FORM P0459

For more details about this annex : see Official Journal of the European Patent Office, No. 12/82

**EP 4 553 846 A1**

**Patent documents cited in the description**

- KR 1020230154385